# EUROPÄISCHE PATENTSCHRIFT

(11) **EP 1 272 812 B1**
(45) Veröffentlichungstag und Bekanntmachung des Hinweises auf die Patenterteilung: **24.11.2004**
(21) Anmeldenummer: 01931382.4
(22) Anmeldetag: 29.03.2001
(51) Int. Cl.: G01B 11/30, G01N 21/47, A61B 5/00

(54) **INTERFEROMETRISCHE MESSVORRICHTUNG**
INTERFEROMETRIC MEASURING DEVICE
DISPOSITIF DE MESURE INTERFEROMETRIQUE

(30) Priorität: 30.03.2000 DE 10015878; 23.12.2000 DE 10065179; 28.03.2001 DE 10115524
(43) Veröffentlichungstag der Anmeldung: 08.01.2003
(73) Patentinhaber: ROBERT BOSCH GMBH, 70442 Stuttgart (DE)
(72) Erfinder: PRINZHAUSEN, Friedrich, 73732 Esslingen (DE); LINDNER, Michael, 71397 Leutenbach (DE)
(74) Vertreter: Fleck, Hermann-Josef, Dr.-Ing.
(86) Internationale Anmeldenummer: PCT/DE2001/001191
(87) Internationale Veröffentlichungsnummer: WO 2001/075395

(56) Entgegenhaltungen:
- WO-A-97/27468
- WO-A-97/32182
- WO-A-99/46557

## Beschreibung

Die Erfindung bezieht sich auf eine interferometrische Messvorrichtung zur Formvermessung einer Oberfläche eines Objekts mittels Weißlichtinterferometrie mit einer eine kurzkohärente Strahlung abgebenden Strahlungsquelle, einem Strahlteiler zum Bilden eines über einen Objektlichtweg zu dem Objekt geleiteten Objektstrahls und eines über einen Referenzlichtweg zu einer reflektierenden Referenzebene geleiteten Referenzstrahls und mit einem Bildaufnehmer, der die von dem Objekt und der Referenzebene zurückgeworfene und zur Interferenz gebrachte Strahlung aufnimmt und einer Auswerteeinrichtung zum Bestimmen der Oberflächenform zuführt, wobei im Objektlichtweg eine bezüglich des Objektes starre Optik angeordnet ist, der nach Reflektion des Objektstrahls eine in Richtung ihrer optischen Achse bewegliche Optik folgt.

### Stand der Technik

Eine derartige interferometrische Messvorrichtung ist in der WO 97/27468 A angegeben. Bei dieser bekannten interferometrischen Messvorrichtung sind in einem Chip eine Vielzahl Interferometer-Module mit jeweiligem Referenzarm und Objektarm und zugeordneter Lichtquelle sowie zugeordnetem Detektor integriert. Mit dieser Messvorrichtung soll eine Probe, insbesondere z.B. die menschliche Haut, großflächig abgetastet werden, wobei die Messpunkte einzelnen Lichtaustrittsöffnungen der Interferometer-Module aus dem Chip zugeordnet und dadurch diskret mit verhältnismäßig großen Abständen lateral über das Objekt verteilt sind. Jedem Interferometer-Modul und damit jedem Messpunkt ist eine eigene Lichtquelle und ein eigener Detektor zugeordnet. Zusätzlich zu der diskreten lateralen Abtastanordnung kann noch ein lateraler Auslenkmechanismus mit mechanischer Bewegung vorgesehen sein. Damit ergibt sich ein relativ aufwendiger Aufbau der interferometrischen Messvorrichtung, die zudem eine ausgedehnte, gut zugängliche Oberfläche zum Anordnen des Messkopfs mit dem Chip erfordert. Zwar kann den einzelnen probeseitigen Austrittsstellen der Interferometer-Modulen zur Vergrößerung eines eingangsseitigen Aperturwinkels α und ausgangsseitigen (objektseitigen) Aperturwinkels β ein zusätzliches optisches Abbildungssystem vorgeschaltet sein, jedoch wird damit der Aufwand der Messvorrichtung noch erhöht. Zur Tiefenabtastung kann der Chip mit dem zusätzlichen optischen Abbildungssystem gegenüber einer bezüglich der Probe ortsfesten Optik in Tiefenrichtung bewegt werden, um die beabsichtigte Volumenabtastung in dem Hautgewebe vorzunehmen.

Bei einer in der WO 99/46557 A gezeigten weiteren interferometrischen Messvorrichtung, die insbesondere ebenfalls zur Abtastung von biologischem Gewebe dient, ist ebenfalls eine relativ zu einem Messobjekt in Tiefenrichtung bewegbare Abtastoptik vorgesehen. Eine laterale Abtastung wird mittels eines Schwingspiegels bewirkt, während mittels eines zugeordneten Detektors die Lichtintensität erfasst und nachfolgend ausgewertet wird.

Eine weitere interferometrische Messvorrichtung ist in der DE 41 08 944 A1 angegeben. Bei dieser bekannten Interferometrischen Messvorrichtung, die auf dem Messprinzip der sogenannten Weisslichtinterferometrie oder Kurzkohärenzinterferometrie beruht, gibt eine Strahlungsquelle kurzkohärente Strahlung ab, die über einen Strahlteiler in einen ein Messobjekt beleuchtenden Objektstrahl und einen eine reflektierende Referenzebene in Form eines Referenzspiegels beleuchtenden Referenzstrahl aufgeteilt wird. Um die Objektoberfläche in Tiefenrichtung abzutasten, wird der Referenzspiegel mittels eines Piezo-Stellelements in Richtung der optischen Achse des Referenzlichtwegs verfahren. Wenn der Objektlichtweg und der Referenzlichtweg übereinstimmen, ergibt sich im Bereich der Kohärenzlänge ein Maximum des Interferenzkontrasts, der mittels eines fotoelektrischen Bildaufnehmers und einer nachgeschalteten Auswerteeinrichtung erkannt und zur Bestimmung der Kontur der Objektoberfläche auf der Grundlage der bekannten Austenkposition des Referenzspiegels ausgewertet wird.

Weitere derartige Interferometrische Messvorrichtungen bzw. Interferometrische Messverfahren auf der Basis der Weisslichtinterferometrie sind in P. de Groot, L. Deck, "Surface profiling by analyis of white-light interferograms in the spatial frequency domain" J. Mod. Opt., Vol. 42, No. 2, 389-401, 1995 und T. Maack, G. Notni, W. Schreiber, W.-D. Prenzel, "Endoskopisches 3-D-Formmesssystem", in Jahrbuch für Optik und Feinmechanik, Ed. W.-D. Prenzel, Verlag Schiele und Schoen, Berlin, 231-240, 1998 angegeben.

Bei den genannten Interferometrischen Messvorrichtungen bzw. Messverfahren besteht eine Schwierigkeit darin, Messungen an unterschiedlichen, insbesondere schwer zugänglichen Stellen, wie z. B. in tiefen Hohlräumen bzw. engen Kanälen mit ausreichender lateraler Auflösung vorzunehmen. In der (nicht vorveröffentlichten) deutschen Patentanmeldung Nr. 199 48 818 ist zum Beheben dieses Problems vorgeschlagen, in dem Arm des Objektlichtwegs mindestens eine Zwischenabbildung zu erzeugen, womit eine größere laterale Auflösung auch bei einem engen Hohlraum oder engen Kanal erreicht wird. Andererseits ergibt sich aus einer Vergrößerung der numerischen Appertur eine geringere Schärfentiefe und auch eine Abtastung eines Oberflächenbereichs, dessen Normale (Blickrichtung) schräg zur Achse der Abbildungsvorrichtung des Objektlichtwegs orientiert ist, führt zu Problemen bei der Abtastung in Tiefenrichtung.

Das Problem, bei der Tiefenabtastung den Bereich der Schärfentiefe einzuhalten, lässt sich dadurch umgehen, dass nicht die Referenzebene bzw. der diese bildende Referenzspiegel bewegt wird, sondern der Referenzlichtweg festgehalten und statt dessen der Objektlichtweg geändert wird. Dies kann wiederum auf zweierlei Art erfolgen, nämlich zum einen dadurch, dass das Objekt selbst in Tiefenrichtung bewegt wird, oder zum ändern dadurch, dass der interferometrische Teil des Messgerätes relativ zum Objekt bewegt wird. Zwar sind derartige Änderungen des Objektlichtwegs als Maßnahme zur Tiefenabtastung in der Weisslichtinterferometrie beispielsweise aus den vorstehend genannten Zeitschriftenartikeln an sich bekannt, jedoch sind sie technisch insbesondere bei der Fertigung von Teilen schwer zu verwirklichen.

Der vorliegenden Erfindung liegt die Aufgabe zugrunde, eine interferometrische Messvorrichtung bereitzustellen, die eine einfache Anpassbarkeit an verschiedene Messprobleme, insbesondere auch eine Messung in engen Hohlräumen gestattet bei relativ großer lateraler Auflösung mit flächiger Auswertung und möglichst einfachem Aufbau und einfacher Messdurchführung.

Diese Aufgabe wird mit den Merkmalen des Anspruches 1 gelöst. Hierbei ist vorgesehen, dass die starre Optik Teil einer ein Zwischenbild der Objektoberfläche erzeugenden Optik ist, und dass der Bildaufnehmer flächig angeordnete Bildaufnahmeelemente aufweist.

Mit der starren Optik und der ihr nachgeschalteten beweglichen Optik ergeben sich vielfältige Möglichkeiten, auf einfache Weise verschiedene Oberflächen auch an schwer zugänglichen Stellen zu vermessen. Beispielsweise ergibt sich mit einem Ablenkelement die Möglichkeit, eine schräg zur Bewegungsrichtung der Tiefenabtastung (Tiefenscan) gerichtete Oberfläche positionsgetreu in Tiefenrichtung abzutasten. Mit anderen, die Wellenfront verformenden Abbildungselementen, wie refraktiven, diffraktiven oder reflektiven Elementen (z.B. Linsen, Konkavspiegel, Gitter etc.) oder einer Kombination derartiger optischer Elemente ergeben sich weitere Anpassmöglichkeiten an ein jeweiliges Messproblem, ohne den Gesamtaufbau der Messvorrichtung aufwendig modifizieren zu müssen. Mit der ein Zwischenbild erzeugenden Optik gelingt es, bei relativ großer lateraler Auflösung einen relativ großen Oberflächenbereich insbesondere auch in engen Hohlräumen auszumessen, da die in das Objekt einzuführende Optik trotz der hohen lateralen Auflösung des relativ großen Messfelds mit sehr geringem Durchmesser aufgebaut werden kann. Dabei kann die örtliche Oberflächenstruktur innerhalb des Messfelds mittels der einzelnen Bildaufnahmeelemente (Pixel) ohne laterale Bewegung praktisch zusammenhängend dreidimensional erfasst werden, wobei lediglich eine Bewegung in Tiefenrichtung (z-Richtung) der beweglichen Optik relativ zu der Objektoberfläche erforderlich ist. Die erfindungsgemäßen Maßnahmen ergeben wesentliche Vorteile. Ist vorgesehen, dass die starre Optik ganz oder teilweise als Endoskop ausgebildet ist, so ergibt sich die Möglichkeit, auch bei einer Messung in engen Hohlräumen eine relativ große laterale Auflösung zu erzielen.

Zum Erreichen einer gegen laterale Relativbewegung des Objektes robusten Messung ist vorteilhaft vorgesehen, dass die starre Optik das Objekt nach Unendlich abbildet.

Verschiedene Aufbauten bestehen darin, dass die bewegliche Optik ganz außerhalb, teilweise innerhalb und außerhalb, oder ganz innerhalb des Objektlichtweges liegt.

Mit den Maßnahmen, dass die bewegliche Optik ganz oder teilweise aus optischen Elementen besteht, die in der optischen Achse beweglich sind, lässt sich z.B. einfach ein Zoom-Objektiv aufbauen.

Zur Genauigkeit der Messung tragen die Maßnahmen bei, dass ein Bild der Referenzebene im Schärfentiefebereich der das Objekt auf dem Bildaufnehmer abbildenden Optik (abbildende Optik) liegt. Hierbei ist es vorteilhaft, dass das Bild der Referenzebene in der Bildebene der abbildenden Optik liegt, und weiterhin, dass sich das Bild der Referenzebene bei Bewegung der beweglichen Optik synchron mit der Bildebene der abbildenden Optik bewegt.

Eine vorteilhafte Ausgestaltung der Erfindung besteht desweiteren darin, dass die starre Optik als starre Zwischenabbildungsvorrichtung ausgebildet ist, mit der mindestens ein zum Objekt starres Zwischenbild der Objektoberfläche - vorzugsweise im Objektlichtweg - erzeugt wird, und dass als bewegliche Optik eine im Strahlengang hinter dem starren Zwischenbild folgende Objektiv-Optik in Richtung ihrer optischen Achse beweglich zur Abtastung des normal zu dieser Achse ausgerichteten starren Zwischenbilds in Tiefenrichtung und Abbilden desselben direkt oder über eine oder mehrere Zwischenabbildungen auf dem Bildaufnehmer ausgebildet ist. Durch die Erzeugung des z.B. im Objektlichtweg liegenden starren Zwischenbilds der Objektoberfläche mit der starren Zwischenbildabbildungsvorrichtung in dem Objektlichtweg wird zum einen auch in engen Kanälen oder Bohrungen die zu messende Objektoberfläche mit relativ großer lateraler Auflösung erfassbar und mit dem Bildaufnehmer und der nachgeschalteten Auswerteeinrichtung hinsichtlich der Tieferistruktur auswertbar. Die Abtastung des starren Zwischenbildes ist mit relativ einfachen Maßnahmen möglich, da zu seiner Tiefenabtastung nur wenige optische Komponenten des Objektlichtwegs bewegt werden müssen, wobei die jeweils abgetastete Tiefe des starren Zwischenbilds stets im Schärfentiefenbereich der beweglichen Objektiv-Optik bleibt, da durch die Tiefenabtastung (Tiefenscan) die Objektebene der bewegten Objektiv-Optik gleichsam durch das starre Zwischenbild hindurch bewegt wird und auf diese Weise die Interferenzmaxima im Bereich größter Schärfe ausgewertet werden. Darüber hinaus ist das starre Zwischenbild stets normal zur Bewegungsrichtung der Objektiv-Optik gerichtet bzw. ausrichtbar, da auch eine bezüglich der Achse des Objektlichtwegs schräge Anordnung der betrachteten Objektoberfläche leicht normal zur Achse der bewegten Objektiv-Optik abbildbar ist. Dadurch lassen sich ohne Weiteres Oberflächenbereiche vermessen, deren Normale schräg zur Bewegungsrichtung der Objektiv-Optik gerichtet ist.

Die Abbildungsqualität und Genauigkeit der Auswertung wird dadurch begünstigt, dass die Zwischenabbildungsvorrichtung einen für alle im Zwischenbild abgebildeten Objektpunkte gleichen Abbildungsmaßstab besitzt. Beispielsweise kann dabei der Aufbau derart sein, dass die Zwischenabbildungsvorrichtung als telezentrische Abbildungsvorrichtung in 4f-Anordnung ausgebildet ist.

Zum Erzielen genauer Messergebnisse ist weiterhin vorteilhaft, dass in dem Referenzlichtweg zur Kompensation eine der Optik im Objektlichtweg zumindest teilweise entsprechende (bzw. identische) Optik vorhanden ist.

Eine für die flächige Auswertung des betrachteten Oberflächenbereichs vorteilhafte Ausbildung der Messvorrichtung besteht darin, dass für jedes Pixel die Position der Objektiv-Optik detektiert wird, bei welcher der höchste Interferenzkontrast auftritt.

Die Tiefenabtastung einer schräg angeordneten Objektoberfläche wird auf einfache Weise dadurch ermöglicht, dass bei einer von der Normalen der Objektoberfläche abweichenden Blickrichtung der Zwischenabbildungsvorrichtung eine Abbildungseinheit zum Erzeugen des Zwischenbilds vorgesehen ist.

Ein einfacher, gut handhabbarer Aufbau der Messvorrichtung wird dadurch begünstigt, dass eine bewegliche Einheit außer der beweglichen Objektiv-Optik, eine Beleuchtungseinheit mit der Lichtquelle und den Strahlteiler oder außer der beweglichen Objekt-Optik nur den Strahlteiler umfasst.

Eine für die Handhabung und den Aufbau günstige Gestaltung der Messvorrichtung besteht weiterhin darin, dass die starre Zwischenabbildungsvorrichtung als Endoskop ausgebildet ist.

Mit den Maßnahmen, dass zur Beleuchtung des Objekts und der Referenzebene eine Faseroptik vorgesehen ist, ergibt sich der Vorteil, dass Reflexionen an den Linsen der Abbildungsvorrichtung reduziert werden.

Verschiedene Ausbildungsmöglichkeiten der Lichtwege ergeben sich dadurch, dass der Objektlichtweg und der Referenzlichtweg sowie weitere Lichtwege als Linsen Achromate, Grin-(Gradient-Index-)Linsen oder Stablinsen aufweisen.

Die Formvermessung wird dadurch begünstigt, dass in der abbildenden Optik innerhalb oder außerhalb des Objektlichtweges sich ein optisches Element befindet, durch welches das Bild in eine für die Auswertung günstige Lage drehbar ist.

Die Zugänglichkeit einer Messstelle innerhalb eines Objekts kann dadurch erleichtert werden, dass in dem Objektlichtweg als starre Optik oder Teil der starren Optik ein Klapp-Endoskop angeordnet ist, das in mindestens zwei Klappstellungen mit einem Winkel zwischen einem eingeklappten und einem ausgeklappten Zustand einstellbar ist.

Dabei ist vorteilhaft vorgesehen, dass das Klapp-Endoskop zwei mit einem Gelenk miteinander verbundene Tuben aufweist, in denen optische Komponenten des Klapp-Endoskops einschließlich eines Ablenkelements untergebracht sind.

Die Klappstellungen lassen sich leicht dadurch einstellen, dass im Bereich des Gelenks ein mit beiden Tuben zusammenwirkender Federmechanismus angeordnet ist.

Die Erfindung wird nachfolgend anhand von Ausführungsbeispielen unter Bezugnahme auf die Zeichnungen näher erläutert. Es zeigen:
- Fig. 1A und 1B: eine schematische Darstellung eines ersten Ausführungsbeispiels einer interferometrischen Messvorrichtung mit einer bezüglich eines Objektes starren Ablenkeinheit in zwei verschiedenen Tiefenabtastpositionen,
- Fig. 2A und 2B: eine Darstellung eines weiteren Ausführungsbeispiels der interferometrischen Messvorrichtung, wobei das Ablenkelement durch abbildende, die Wellenfront verformende Elemente ersetzt ist, in zwei verschiedenen Abtastpositionen,
- Fig. 3: ein weiteres Ausführungsbeispiel der interferometrischen Messvorrichtung, mit einer anderen Anordnung der optisch starren Elemente im Objektlichtweg,
- Fig. 4A und 4B: ein weiteres Ausführungsbeispiel der interferometrischen Messvorrichtung, bei dem die Größe einer bewegten Einheit des Referenzlichtweges verkleinert ist,
- Fig. 5A und 5B: ein weiteres Ausführungsbeispiel, bei dem sowohl bewegliche Optik als auch der Referenzspiegel bewegt werden,
- Fig. 6: eine schematische Darstellung eines weiteren Ausführungsbeispiels einer interferometrischen Messvorrichtung,
- Fig. 7: eine Darstellung der interferometrischen Messvorrichtung nach Fig. 6 mit einer Anordnung zur Vermessung einer schrägen Objektoberfläche,
- Fig. 8: ein weiteres Beispiel der interferometrischen Messvorrichtung, bei der die Anzahl bewegter Elemente gegenüber den Fig. 6 und 7 verringert ist,
- Fig. 9: ein weiteres Ausführungsbeispiel der interferometrischen Messvorrichtung, bei der die Anzahl bewegter Elemente weiterhin verringert ist,
- Fig. 10: eine weitere Ausführungsform der interferometrischen Messvorrichtung, bei der zur Beleuchtung eine Lichtleitphaseranordnung vorgesehen ist,
- Fig. 11: eine weitere Ausführungsform der interferometrischen Messvorrichtung mit einer Einrichtung zum Drehen des Bildes,
- Fig. 12: eine Ausgestaltung der interferometrischen Messvorrichtung mit einem Klapp-Endoskop während eines Einführvorganges,
- Fig. 13: die Messvorrichtung nach Fig. 12 in Messposition und
- Fig. 14a) bis c): verschiedene Darstellungen des Klapp-Endoskops.

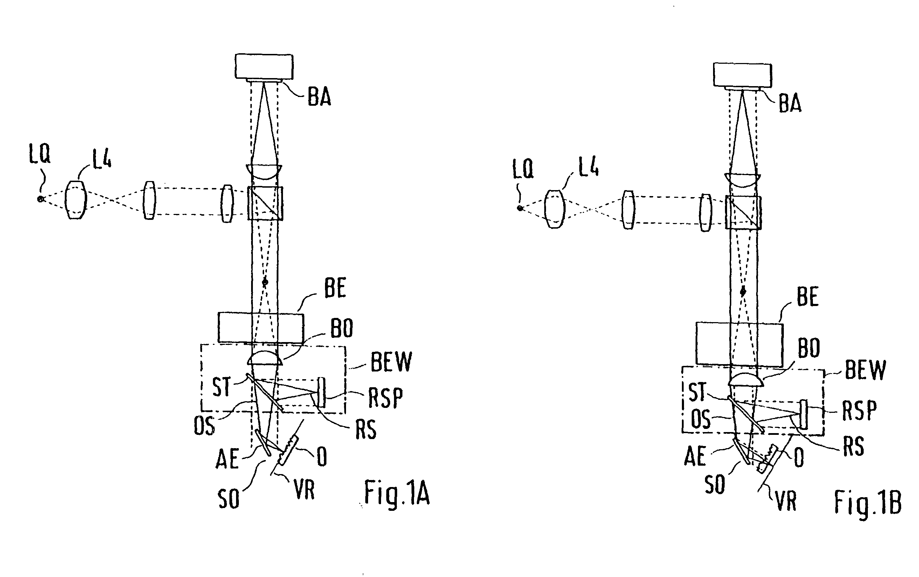

Bei einer in Fig. 1 A und 1 B gezeigten interferometrischen Messvorrichtung 1 wird kurzkohärente Strahlung einer Strahlungsquelle bzw. Lichtquelle LQ (z.B. einer Leuchtdiode oder Superlumineszenzdiode), deren Kohärenzlänge typischerweise in der Größenordnung von ca. 10 µm (z.B. 3 bis 100 µm) liegt, über eine Linse L4 und weitere optische Elemente auf einen Strahlteiler ST geführt und in diesem in einen zu einer Oberfläche eines Objekts O geleiteten Objektstrahl OS und einen zu einem Referenzspiegel RSP geleiteten Referenzstrahl RS aufgeteilt.

In dem Objektlichtweg bzw. Objektarm ist eine starre Optik SO in Form einer reflektierenden Ablenkeinheit AE vor dem Objekt O in der Weise angeordnet, dass das schräg gestellte Objekt O stets senkrecht zu seiner Oberfläche in Tiefenrichtung abgetastet wird, wie die Fig. 1A und 1 B erkennen lassen, in denen zwei verschiedene Abtasttiefen dargestellt sind, die durch die Auslenkung einer bewegten Einheit BEW mittels eines daran angebrachten Bewegungserzeugers BE, beispielsweise eines Piezoelementes bewirkt werden. Eine virtuelle Referenzebene VR liegt dadurch in der Normalenrichtung in verschiedenen Tiefen bezüglich des Objektes O. Die starre Optik SO, die vorliegend durch die Ablenkeinheit AE gebildet wird, ist starr bezüglich des Objektes O. Ihr folgt im Strahlengang eine bewegliche Optik BO, die in der bewegten Einheit BEW in dem Strahlengang zwischen dem Strahlteiler ST und dem Bildaufnehmer A angeordnet ist.

Wenn bei der Tiefenabtastung durch die Bewegung der bewegten Einheit BEW der Objektlichtweg und der Referenzlichtweg übereinstimmen, ergibt sich im Bereich der Kohärenzlänge ein Maximum des Interferenzkontrasts, der mittels des photoelektrischen Bildaufnehmers BA und einer nachgeschalteten Auswerteeinrichtung erkannt und zur Bestimmung der Kontur der Objektoberfläche auf der Grundlage der bekannten Austenkposition ausgewertet wird.

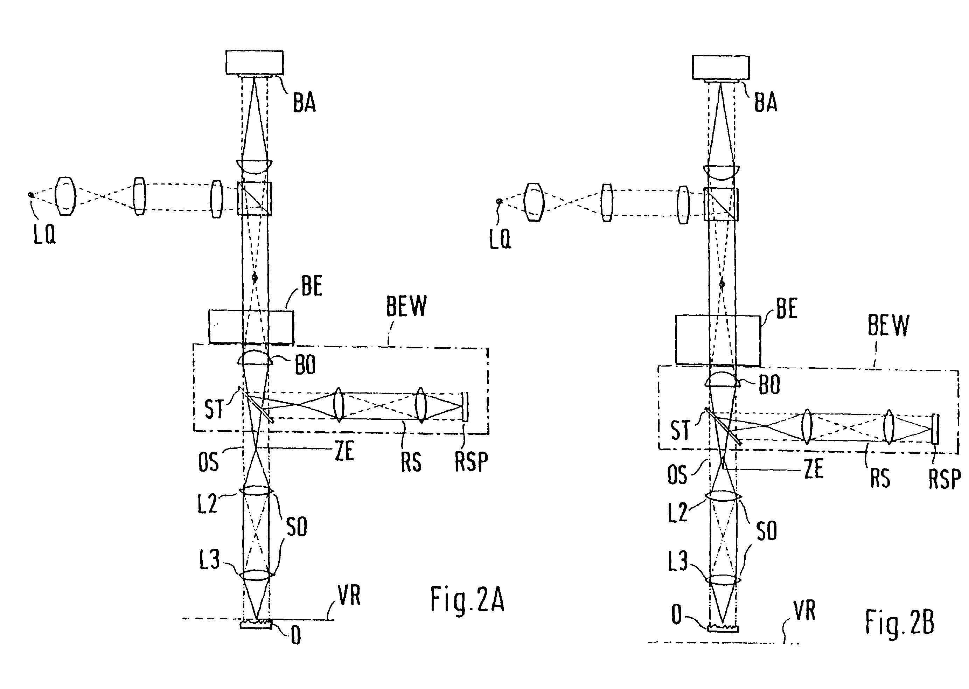

Bei dem in den Fig. 2A und 2B gezeigten weiteren Ausführungsbeispiel, in denen wiederum zwei unterschiedliche Auslenkpositionen der bewegten Einheit BEW mittels des Bewegungserzeugers BE dargestellt sind, ist gegenüber dem vorhergehenden Ausführungsbeispiel die starre Optik SO anders ausgebildet, nämlich durch abbildende, die Wellenfront verformende Elemente in Form von Linsen L2, L3. Mit der starren Optik SO wird ein starres Zwischenbild in einer Zwischenbildebene ZE erzeugt, das mittels der in der bewegten Einheit BEW befindlichen bewegten Optik in Tiefenrichtung abgetastet wird. Mit dieser Maßnahme ergibt sich eine einfache Anpassungsmöglichkeit einer an sich gleichbleibenden Abtasteinheit an unterschiedliche Messsituationen, wobei z.B. eine Messung in engen Hohlräumen bei relativ großer lateraler Auflösung erreicht wird. Auch ergibt sich kein Problem hinsichtlich der Schärfentiefe, da die Objektoberfläche stets optimal bezüglich der abbildenden beweglichen Optik BO ausgerichtet ist. Das Objekt O wird über die abbildende Optik, die die starre Optik SO und die bewegliche Optik BO enthält, auf dem Bildaufnehmer BA abgebildet. Durch geeignete Anordnung der Komponenten in der bewegten Einheit BEW kann erreicht werden, dass das Bild der Referenzebene VR mit der Bildebene der abbildenden Optik bewegt wird.

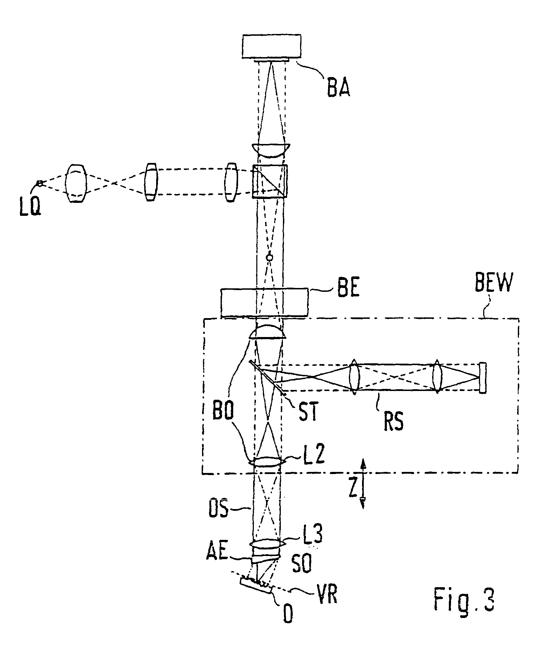

Bei einem in Fig. 3 gezeigten weiteren Ausführungsbeispiel umfasst die starre Optik SO ein abbildendes, die Wellenfront veränderndes Element in Form der Linse L3 und eine Ablenkeinheit in Form eines brechenden Elementes, so dass wiederum eine bezüglich der Tiefenabtastrichtung schräg liegende Objektoberfläche stets positionsgetreu vermessen wird. Ein weiteres, die Wellenfront veränderndes Element in Form der Linse L2 liegt hierbei jedoch innerhalb der bewegten Einheit BEW, so dass sich hierbei zwar ebenfalls eine einfache Anpassungsmöglichkeit an eine Abtastvorrichtung ergibt, andererseits aber der Tiefenmessbereich auf die Schärfentiefe eingeschränkt ist, da die Gesamtbildebene nicht mit der Tiefenabtastung mit wandert. Die starre Optik SO bildet das Objekt nach unendlich ab.

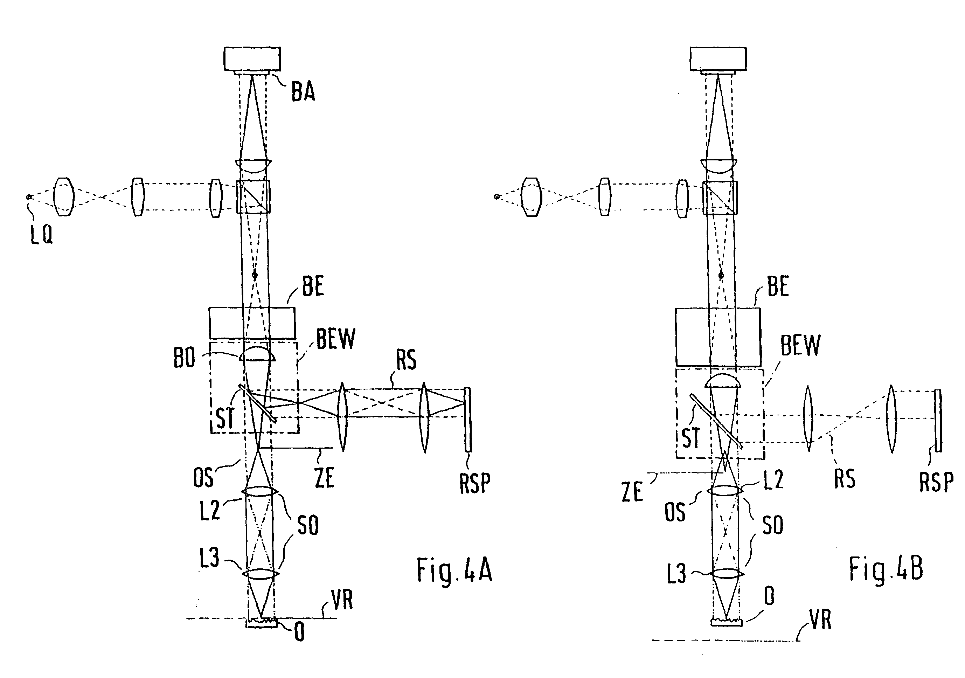

Bei dem in den Fig. 4A und 4B ebenfalls in zwei verschiedenen Abtaststellungen gezeigten Ausführungsbeispiel ist die bewegte Einheit BEW sehr klein ausgebildet, wobei ein größerer Durchmesser von Abbildungslinsen im Referenzlichtweg in Verbindung mit der Strahlverteilung auf dem Strahlteiler ST genutzt wird.

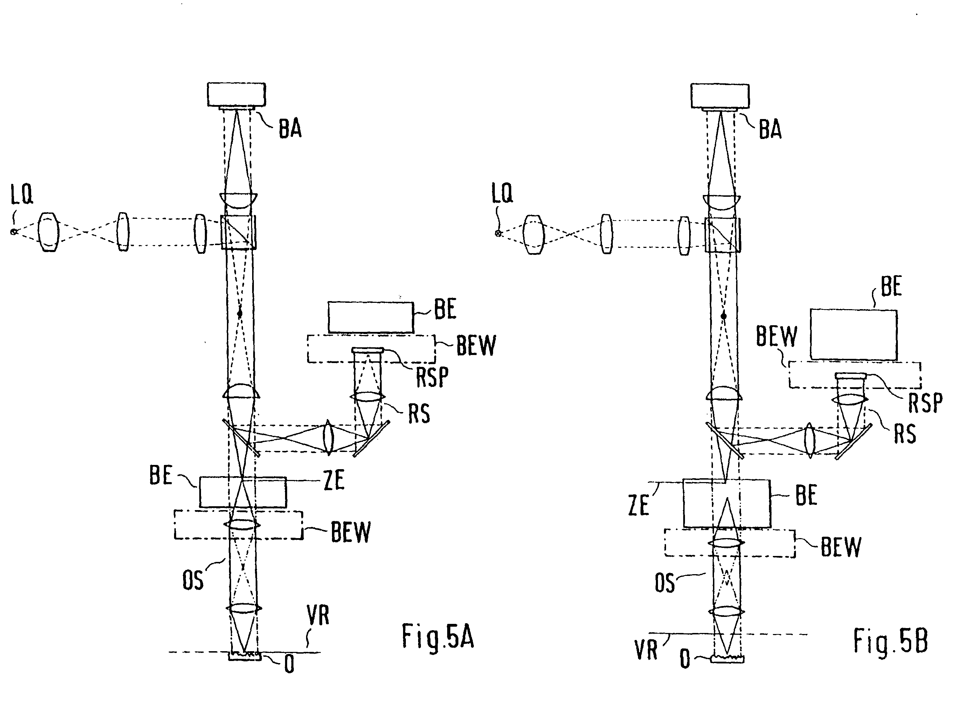

Bei einem in den Fig. 5A und 5B gezeigten weiteren Ausführungsbeispiel sind jeweils eine bewegte Einheit BEW im Referenzlichtweg und im Objektlichtweg vorgesehen, die synchron mittels eines jeweils zugeordneten Bewegungserzeugers BE ausgelenkt werden.

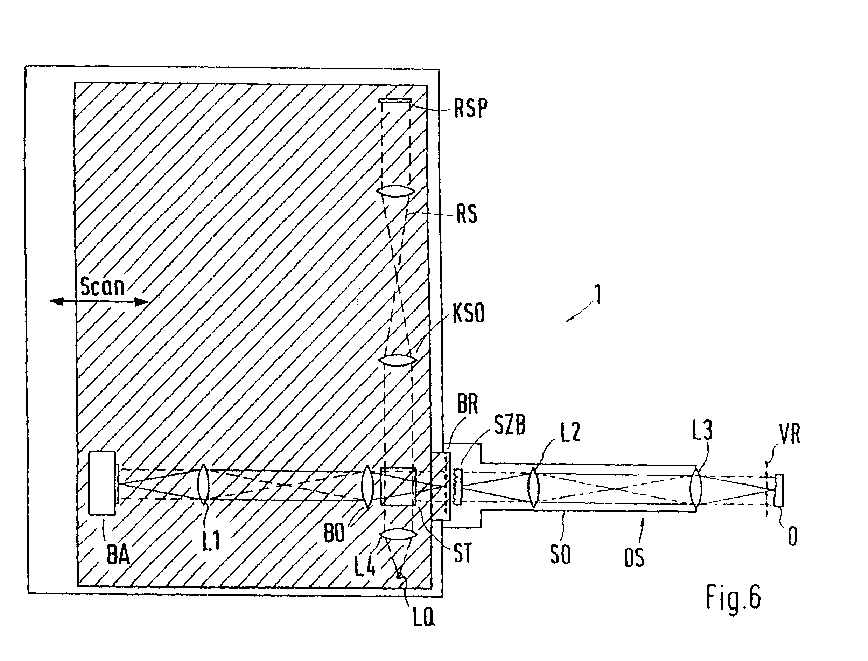

Weitere bevorzugte Ausführungsbeispiele sind in den Fig. 6 bis 10 gezeigt. Gemäß dem Ausführungsbeispiel nach Fig. 6 ist in dem Objektlichtweg bzw. Objektarm als starre Optik SO eine bezüglich des Objektes O starre Zwischenabbildungsvorrichtung SO (im Folgenden auch als Bajonett-Optik bezeichnet) mit Zwischenabbildungs-Linsen L2, L3 angeordnet, mit der ein starres Zwischenbild SZB der Objektoberfläche erzeugt wird. Der Referenzlichtweg entspricht in seiner Länge dem Objektlichtweg, so dass eine virtuelle Referenz VR im Bereich der Objektoberfläche liegt. Ein Bild der virtuellen Referenz VR wird als Bild der Referenzebene BR im Bereich des starren Zwischenbilds SZB mit der Bajonett-Optik SO erzeugt. Die Bajonett-Optik SO ist für einen günstigen Aufbau und eine günstige Handhabung endoskopartig ausgebildet und beispielsweise an einem das übrige optische System enthaltenden Gehäuse angesetzt. Eine andere Möglichkeit besteht darin, dass die Bajonett-Optik SO von dem Gehäuse mechanisch getrennt und an dem Objekt O ortsfest angekoppelt ist.

Der Referenzlichtweg bzw. Referenzarm enthält den optischen Elementen des Objektarms im Wesentlichen entsprechende optische Elemente als kompensierte Optik KSO, so dass störende optische Eigenschaften der Optik im Objektlichtweg kompensiert werden.

Das von der Bajonett-Optik SO erzeugte starre Zwischenbild SZB wird in Tiefenrichtung, d.h. parallel zu seiner Normalen mittels der parallel zur Normalenrichtung, d.h. entlang ihrer optischen Achse beweglichen Optik BO in Tiefenrichtung abgetastet (Tiefenscan). Das Bild der Referenzebene BR liegt im Schärfentiefenbereich des beweglichen Objektivs, vorzugsweise in der Objektebene des beweglichen Objektivs bzw. der beweglichen Objektiv-Optik. Die Tiefenabtastung erfolgt, indem das bewegliche Objektiv BO relativ zu dem starren Zwischenbild SZB bewegt wird, wobei gewährleistet ist, dass sich das Bild der Referenzebene BR synchron mit der beweglichen Objektiv-Optik BO bewegt.

Dadurch wird mit der Abtastung des beweglichen Objektivs BO das Bild der Referenzebene BR durch das starre Zwischenbild SZB bewegt.

Das starre Zwischenbild SZB wird von der beweglichen Objektiv-Optik BO direkt oder über mindestens eine Zwischenabbildung auf einen eine Vielzahl von nebeneinander liegenden Bildaufnahmeelementen aufweisenden Bildaufnehmer BA, z.B. eine CCD-Kamera abgebildet und in einer nachfolgenden Auswerteeinrichtung zum Bestimmen der Oberflächenform z.B. durch Detektieren der Maxima des Interferenzkontrastes ausgewertet, wobei die jeweilige Position des beweglichen Objektivs BO zugrunde gelegt wird.

Im Bild des Objektes auf dem Bildaufnehmer BA tritt hoher Interferenzkontrast dann auf, wenn ein Gangunterschied in dem Objektarm und dem Referenzarm kleiner als die Kohärenzlänge ist. Zur Gewinnung eines 3D-Höhenprofils können verschiedene, an sich bekannte Verfahren (vgl. die eingangs genannten Druckschriften) angewendet werden.

Der in Fig. 6 und auch den nachfolgenden Fig. gezeigte Aufbau beinhaltet beispielhaft ein Michelson-Interferometer. Mit der starren Bajonett-Optik SO können auch mehrere Zwischenabbildungen erzeugt werden. Für die Abtastung wird der strichliert dargestellte Bereich bewegt, der z.B. innerhalb eines Gehäuses liegen kann, auf das die Bajonett-Optik SO aufgesetzt ist. Alternativ kann die Bajonett-Optik SO auch von dem Gehäuse getrennt und mit dem Objekt ortsfest verbunden sein.

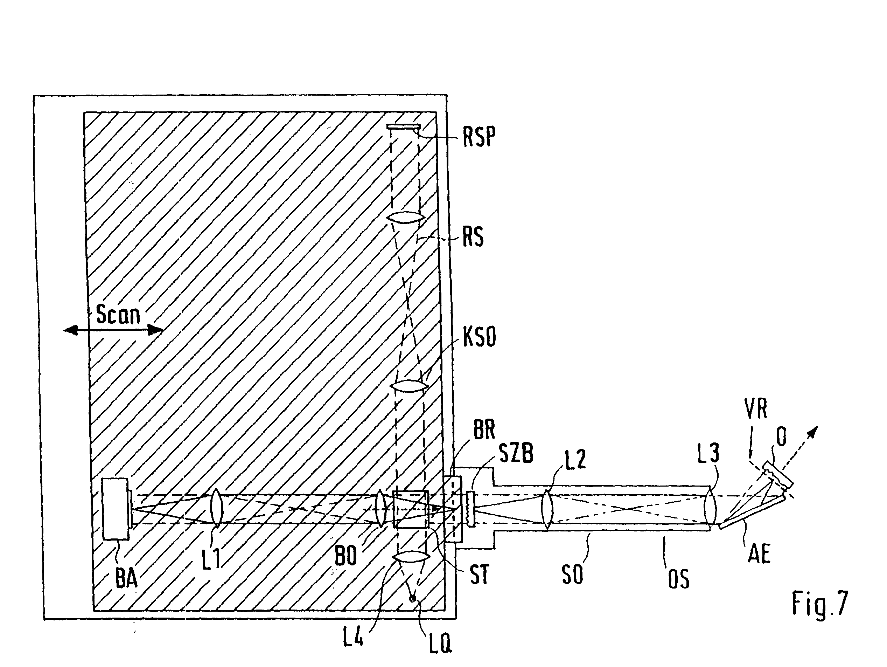

Bei dem in Fig. 7 gezeigten Aufbau ist die zu vermessende Oberfläche des Objekts O bezüglich der optischen Achse der Bajonett-Optik SO schräg angeordnet und vor dem Objekt ein Ablenkelement AE oder eine andere Abbildungseinheit positioniert, über das ein bezüglich der optischen Achse des beweglichen Objektivs BO normal ausgerichtetes starres Zwischenbild SZB erzeugt wird. Über die Abtastung des starren Zwischenbilds SZB ist die Abtastung der schrägen Objektoberfläche mit einfachen Maßnahmen durchführbar, da die Blickrichtung der optischen Achse des beweglichen Objektivs BO auf das Zwischenbild 0° besitzt. Die Abtast-Achse muss nun nur parallel zur Achse des beweglichen Objektivs BO gerichtet sein. Damit ist die Blickrichtung der Bajonett-Optik SO unabhängig von der Abtast-Achse der Tiefenabtastung.

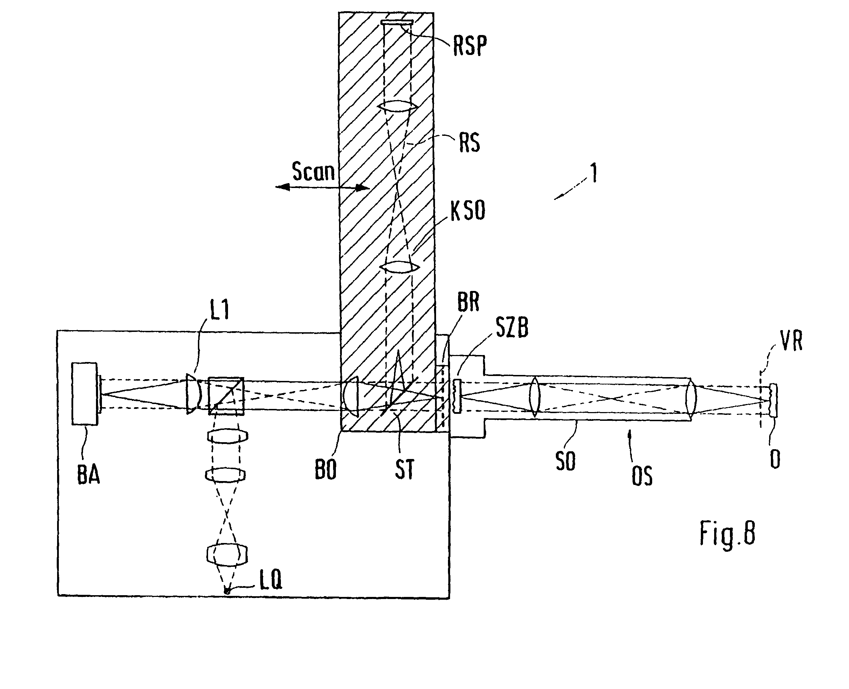

Bei dem in Fig. 8 gezeigten Aufbau der interferometrischen Messvorrichtung 1 ist die Anzahl der bewegten Komponenten, die die Tiefenabtastung ausführen, wesentlich reduziert, wie die Anzahl der in dem strichlierten Bereich dargestellten Elemente zeigt, die im Wesentlichen den Referenzarm, den Strahlteiler ST sowie das bewegliche Objektiv BO umfasst.

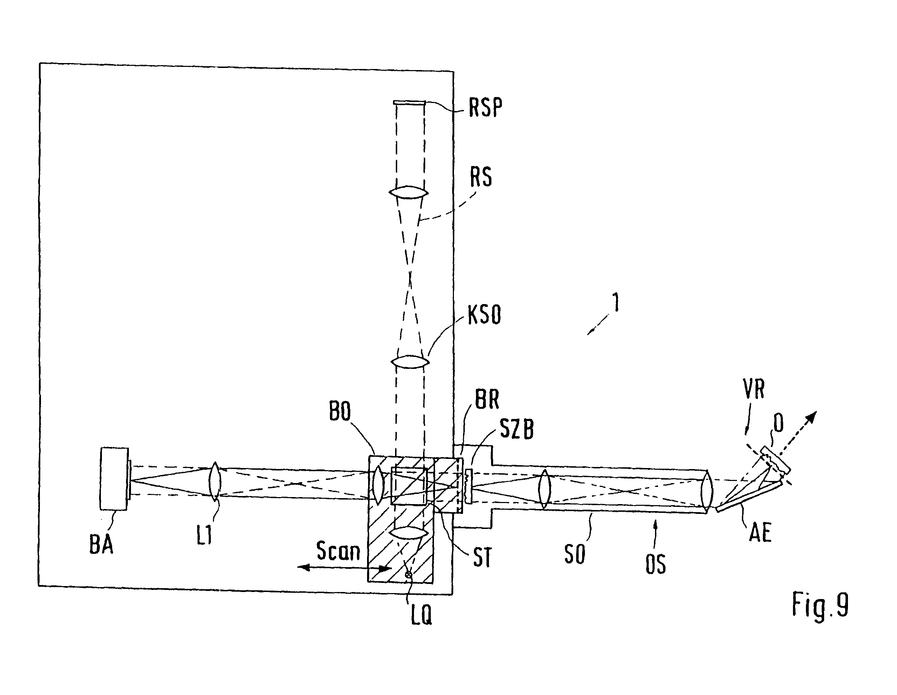

Ein weiteres Ausführungsbeispiel für die interferometrische Messvorrichtung 1 mit Abtastung des starren Zwischenbilds SZB ist in Fig. 9 wiedergegeben. Hierbei wird außer dem beweglichen Objektiv BO der Strahlteiler ST und die Beleuchtungseinheit mit der Lichtquelle LQ bewegt. Eine geringe Verschiebung des Referenzstrahls quer zur optischen Achse des Referenzarms wirkt sich wegen der relativ geringen Abtast-Bewegungsstrecke auf das Messergebnis praktisch nicht aus.

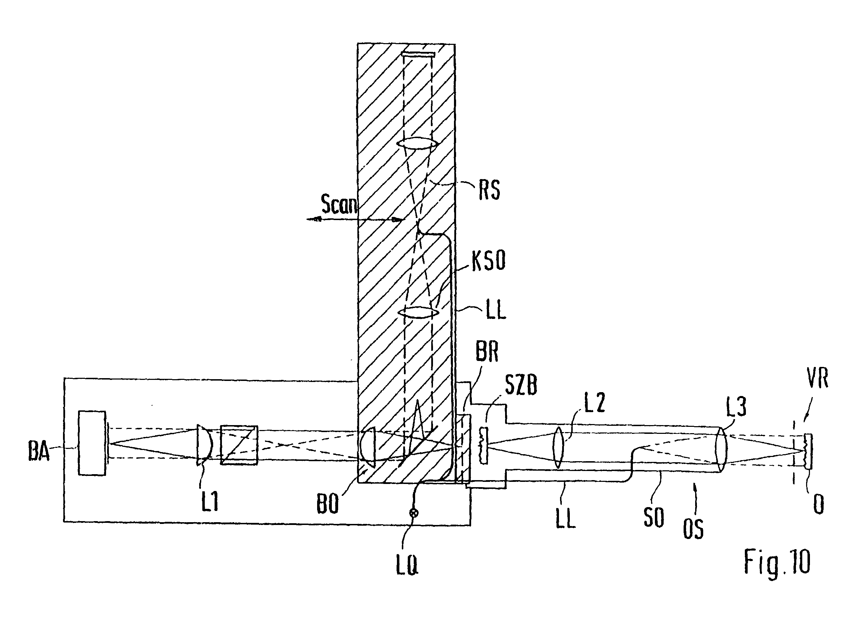

Bei dem in Fig. 10 gezeigten Ausführungsbeispiel wird das Objekt 0 alternativ über eine Faseroptik mit Lichtleitern LL beleuchtet, die zumindest zum Teil innerhalb der Bajonett-Optik SO verläuft. Diese faseroptische Beleuchtung hat den Vorteil, dass Reflexionen an den Linsen der Bajonett-Optik SO reduziert werden. Zum Abgleich der optischen Weglängen und der Dispersion in dem Objektarm und dem Referenzarm sollten die Faserlängen und Geometrien in den beiden Interferometerarmen möglichst übereinstimmend gewählt werden.

Für die in der interferometrischen Messvorrichtung enthaltenen Linsen können verschiedene Ausbildungsformen gewählt werden, z.B. Achromate, Grin-(Gradient-Index-)Linsen oder Stablinsen.

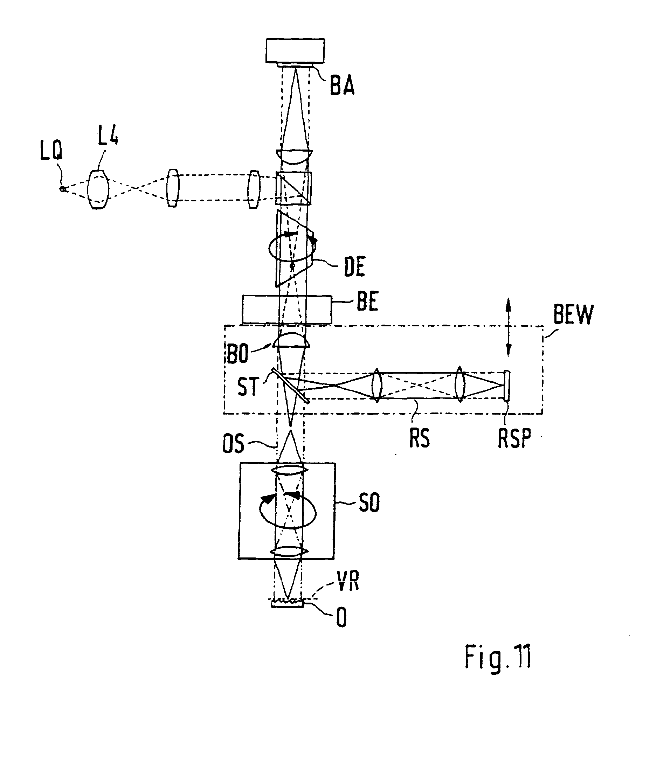

In dem in Fig. 11 gezeigten Ausführungsbeispiel ist in der abbildenden Optik ein optisches Element DE zum Drehen des Bildes enthalten. Wird die starre Optik SO bezüglich des Objektes gedreht, z.B. zur Messung verschiedener Segmente eines radialsymmetrischen Objektes (etwa ein Ventilsitz), so dreht sich auf dem Bildaufnehmer BA ebenfalls das Bild des Objektes O. Es ist jedoch vorteilhaft, auf dem Bildaufnehmer BA ein festes Bild des Objektes zu haben. Dies kann erreicht werden, indem in der abbildenden Optik, vorzugsweise außerhalb des Objektlichtweges, ein optisches Element (z.B. Reversions-, Dove-Prisma, etc.) vorgesehen ist, durch welches die Drehung des Bildes wieder ausgeglichen werden kann.

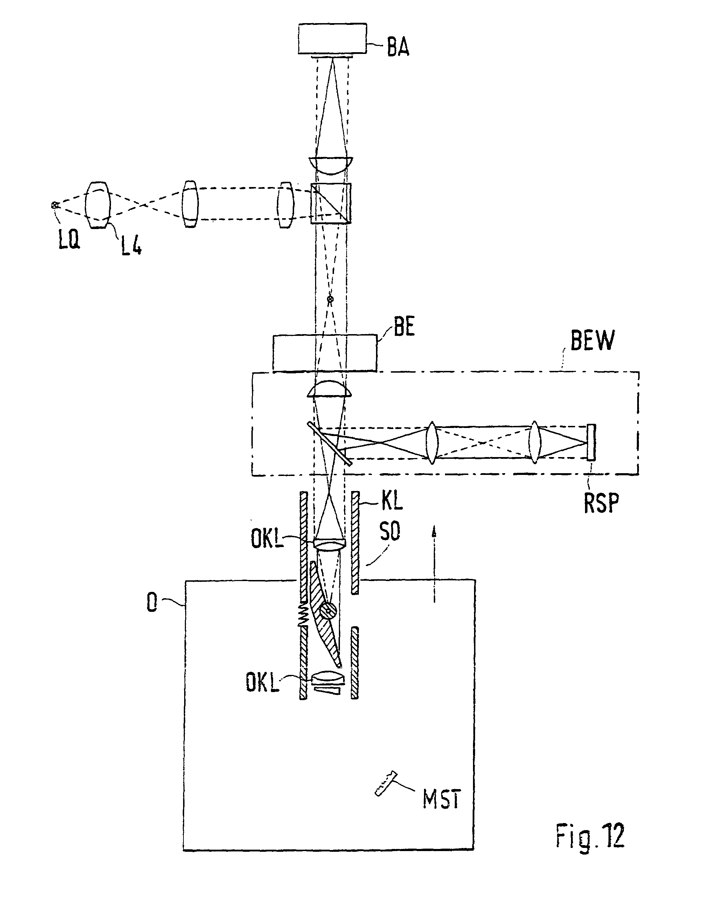

In den Fig. 12 bis 14 ist ein weiteres Ausführungsbeispiel der interferometrischen Messvorrichtung dargestellt, wobei die starre Optik SO als ein Klapp-Endoskop KL ausgeführt ist.

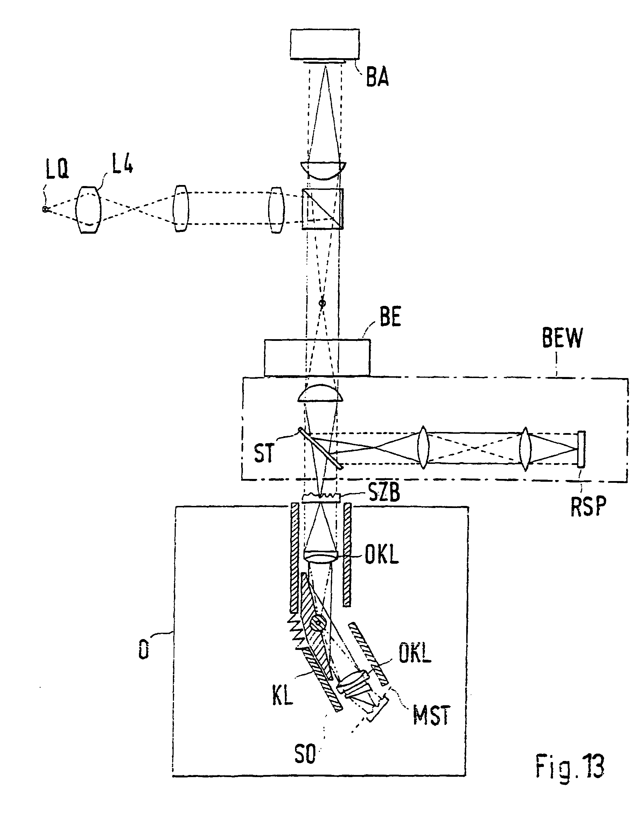

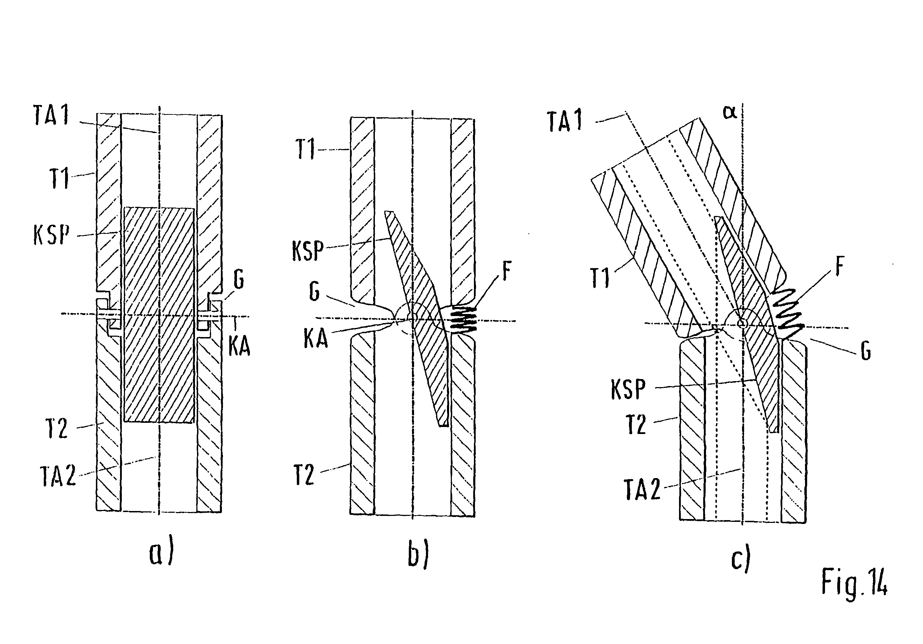

Das Klapp-Endoskop KL besteht beispielsweise aus zwei Tuben T1, T2, die jeweilige Tubusachsen TA1, TA2 aufweisen und mit einem Gelenk G miteinander verbunden sind, um eine Schwenkachse KA des einen Tubus T1 relativ zu dem anderen Tubus T2 zu bilden. Die beiden Tuben T1, T2 können beispielsweise zwei verschiedene Klappstellungen einnehmen, die sich um einen Klappwinkel *a* unterscheiden, wie aus den Fig. 14b) und c) ersichtlich. Vorliegend sind die beiden Tuben T1, T2 mechanisch so gefertigt, dass die beiden Tubusachsen TA1, TA2 im eingeklappten Zustand einen Winkel von 0° bilden, während sie im ausgeklappten Zustand um den vorgegebenen Klappwinkel *a* zueinander orientiert sind. Zwischen den Tuben T1, T2, die die optischen Komponenten OKL des Klapp-Endoskops in sich aufnehmen, befindet sich im Bereich des Gelenkes G ein Federmechanismus mit einer Feder F. Ist das Klapp-Endoskop KL im eingeklappten Zustand, so ist die Feder F gespannt, während sie im ausgeklappten Zustand entspannt ist. Die Endoskop-Optik ist vorliegend für den ausgeklappten Zustand ausgelegt. Die Endoskop-Optik enthält mindestens ein optisches Ablenkelement, z.B. einen Spiegel KSP, der im Bereich des Gelenkes G angeordnet ist. Als Ablenkelemente sind auch Prismen oder Gitter denkbar, durch welche die optische Achse entsprechend den Tubusachsen TA1, TA2 umgelenkt wird. Wie Fig. 12 zeigt, ist bei der Einführung in das Objekt O das Klapp-Endoskop KL im eingeklappten Zustand. Dies kann bei gespannter Feder durch eine eigene Führung des Endoskops erreicht werden. Als Führung kann aber auch das Objekt selbst dienen, z.B. eine Führungsbohrung bei einer Ventitsitzmessung, wie die Fig. 12 und 13 zeigen. Ist das Klapp-Endoskop KL vollständig in das Objekt O bzw. das Bauteil eingeführt, soll das Gelenk G frei liegen, so dass das Klapp-Endoskop KL den ausgeklappten Zustand einnehmen kann. Das Klapp-Endoskop KL ist so gefertigt, dass im ausgeklappten Zustand genau die Messstelle MST beobachtet wird.

Die zu beobachtende Objektoberfläche wird durch das Klapp-Endoskop KL im ausgeklappten Zustand mit einer ebenen Welle beleuchtet und direkt oder über ein Zwischenbild auf den Bildaufnehmer BA (z.B. CCD-Kamera) abgebildet. Im Referenzlichtweg wird der Referenzstrahl RS von dem Referenzspiegel RSP reflektiert. Zur Kompensation der Endoskopoptik kann auch hier in dem Referenzlichtweg bzw. Referenzarm eine der Endoskop-Optik ähnliche oder entsprechende Optik eingesetzt werden. Die Datenauswertung erfolgt wie im Zusammenhang mit den vorangehenden Ausführungsbeispielen beschrieben.

Das Interferometer kann auch hierbei anders als ein Michelson-Interferometer realisiert werden (z.B. als Common-Path, Mach-Zehnder, etc.).

## Patentansprüche

1. Interferometrische Messvorrichtung (1) zur Formvermessung einer Oberfläche eines Objekts (O) mittels Weißlichtinterferometrie mit einer eine kurzkohärente Strahlung abgebenden Strahlungsquelle (LQ), einem Strahlteiler (ST) zum Bilden eines über einen Objektlichtweg zu dem Objekt (O) geleiteten Objektstrahls (OS) und eines über einen Referenzlichtweg zu einer reflektierenden Referenzebene (RSP) geleiteten Referenzstrahls (RS) und mit einem Bildaufnehmer (BA), der die von dem Objekt (O) und der Referenzebene (RSP) zurück geworfene und zur Interferenz gebrachte Strahlung aufnimmt und einer Auswerteeinrichtung zum Bestimmen der Oberflächenform zuführt, wobei im Objektlichtweg eine bezüglich des Objektes (O) starre Optik (SO) angeordnet ist, der nach Reflektion des Objektstrahls (OS) eine in Richtung ihrer optischen Achse bewegliche Optik (BO) folgt,
**dadurch gekennzeichnet,**
**dass** die starre Optik (SO) Teil einer ein Zwischenbild der Objektoberfläche erzeugenden Optik ist, und
**dass** der Bildaufnehmer (BA) flächig angeordnete Bildaufnahmeelemente (Pixels) aufweist.

2. Messvorrichtung nach Anspruch 1,
**dadurch gekennzeichnet,**
**dass** die starre Optik (SO) die Wellenfront verformende Elemente aufweist.

3. Messvorrichtung nach Anspruch 1 oder 2,
**dadurch gekennzeichnet,**
**dass** die starre Optik (SO) ganz oder teilweise als Endoskop ausgebildet ist.

4. Messvorrichtung nach einem der vorhergehenden Ansprüche,
**dadurch gekennzeichnet,**
**dass** die starre Optik (SO) das Objekt nach Unendlich abbildet.

5. Messvorrichtung nach einem der vorhergehenden Ansprüche,
**dadurch gekennzeichnet,**
**dass** die bewegliche Optik (BO) ganz oder teilweise aus optischen Elementen besteht, die in der optischen Achse beweglich gelagert sind.

6. Messvorrichtung nach einem der vorhergehenden Ansprüche,
**dadurch gekennzeichnet,**
**dass** ein Bild der Referenzebene (VR) im Schärfentiefebereich der abbildenden Optik liegt.

7. Messvorrichtung nach Anspruch 6,
**dadurch gekennzeichnet,**
**dass** das Bild der Referenzebene (VR) in der Bildebene der abbildenden Optik liegt.

8. Messvorrichtung nach Anspruch 6 oder 7,
**dadurch gekennzeichnet,**
**dass** sich das Bild der Referenzebene (VR) bei Bewegung der beweglichen Optik (BO) synchron mit der Bildebene der abbildenden Optik bewegt.

9. Messvorrichtung nach einem der vorhergehenden Ansprüche,
**dadurch gekennzeichnet,**
**dass** die starre Optik (SO) als starre Zwischenabbildungsvorrichtung (L2, L3) ausgebildet ist, mit der mindestens ein zum Objekt (O) starres Zwischenbild (SZB) der Objektoberfläche erzeugt wird, und
**dass** als bewegliche Optik (BO) eine im Strahlengang hinter dem starren Zwischenbild (SZB) folgende Objektiv-Optik in Richtung ihrer optischen Achse (ROA) beweglich zur Abtastung des normal zu dieser Achse ausgerichteten starren Zwischenbilds (SZB) in Tiefenrichtung (Z) und Abbilden desselben direkt oder über eine oder mehrere Zwischenabbildungen auf dem Bildaufnehmer (BA) ausgebildet ist.

10. Messvorrichtung nach Anspruch 9,
**dadurch gekennzeichnet,**
**dass** die Zwischenabbildungsvorrichtung (L2, L3) einen für alle im Zwischenbild (SZB) abgebildeten Objektpunkte gleichen Abbildungsmaßstab besitzt.

11. Messvorrichtung nach Anspruch 10,
**dadurch gekennzeichnet,**
**dass** die Zwischenabbildungsvorrichtung (L2, L3) als telezentrische Abbildungsvorrichtung in 4f-Anordnung ausgebildet ist.

12. Messvorrichtung nach einem der vorhergehenden Ansprüche,
**dadurch gekennzeichnet,**
**dass** in dem Referenzlichtweg zur Kompensation eine der Optik im Objektlichtweg zumindest teilweise entsprechende Optik (KSO) vorhanden ist.

13. Messvorrichtung nach einem der vorhergehenden Ansprüche,
**dadurch gekennzeichnet,**
**dass** für jedes Pixel die Position der Objektiv-Optik (BO) detektiert wird, bei welcher der höchste Interferenzkontrast auftritt.

14. Messvorrichtung nach einem der Ansprüche 9 bis 13,
**dadurch gekennzeichnet,**
**dass** bei einer von der Normalen der Objektoberfläche abweichenden Blickrichtung der Zwischenabbildungsvorrichtung (L2, L3) eine Ablenkeinheit (AE) zum Erzeugen des Zwischenbilds (SZB) vorgesehen ist.

15. Messvorrichtung nach einem der vorhergehenden Ansprüche,
**dadurch gekennzeichnet,**
**dass** eine bewegliche Einheit außer der beweglichen Objektiv-Optik (BO), eine Beleuchtungseinheit mit der Lichtquelle (L2) und den Strahlteiler (ST) oder außer der beweglichen Objektiv-Optik (Bo) nur den Strahlteiler (ST) umfasst.

16. Messvorrichtung nach einem der Ansprüche 9 bis 15,
**dadurch gekennzeichnet,**
**dass** die starre Zwischenabbildungsvorrichtung (L2, L3) als Endoskop ausgebildet ist.

17. Messvorrichtung nach einem der vorhergehenden Ansprüche,
**dadurch gekennzeichnet,**
**dass** zur Beleuchtung des Objekts (O) und der Referenzebene (RSP) eine Faseroptik (LL) vorgesehen ist.

18. Messvorrichtung nach einem der vorhergehenden Ansprüche,
**dadurch gekennzeichnet,**
**dass** der Objektlichtweg und der Referenzlichtweg sowie weitere Lichtwege als Linsen (L1, L2, L3, L4, KSO) Einzeilinsen, Grin-(Gradient-Index-)Linsen, Stablinsen, diffraktive Elemente, Prismen oder deren Kombinationen aufweisen.

19. Messvorrichtung nach einem der vorhergehenden Ansprüche,
**dadurch gekennzeichnet,**
**dass** in der abbildenden Optik innerhalb oder außerhalb des Objektlichtweges sich ein optisches Element (DE) zum Drehen eines Bildes des Objekts (O) befindet, durch welches ein festes Bild des Objekts (O) auf dem Bildaufnehmer (BA) erhalten wird, wenn die starre Optik (SO) gedreht wird.

20. Messvorrichtung nach einem der vorhergehenden Ansprüche,
**dadurch gekennzeichnet,**
**dass** in dem Objektlichtweg als starre Optik (SO) oder Teil der starren Optik (SO) ein Klapp-Endoskop (KL) angeordnet ist, das in mindestens zwei Klappstellungen mit einem Winkel (*a*) zwischen einem eingeklappten und einem ausgeklappten Zustand einstellbar ist.

21. Messvorrichtung nach Anspruch 20,
**dadurch gekennzeichnet,**
**dass** das Klapp-Endoskop (KL) zwei mit einem Gelenk (G) miteinander verbundene Tuben (T1, T2) aufweist, in denen optische Komponenten (OKL) des Klapp-Endoskops (KL) einschließlich eines Ablenkelements (KSP) untergebracht sind.

22. Messvorrichtung nach Anspruch 21,
**dadurch gekennzeichnet,**
**dass** im Bereich des Gelenks (G) ein mit beiden Tuben (T1, T2) zusammenwirkender Federmechanismus (F) angeordnet ist.

## Claims

1. Interferometric measurement apparatus (1) for measurement of the shape of a surface of an object (O) by means of white-light interferometry by means of a radiation source (LQ) which emits short-coherent radiation, a beam splitter (ST) for formation of an object beam (OS) which is passed over an object light path to the object (O), and a reference beam (RS) which is passed via a reference light path to a reflective reference plane (RSP), and having an image sensor (BA), which receives the radiation that is thrown back from the object (O) and from the reference plane (RSP) and which leads to interference, and supplies this radiation to an evaluation device for determination of the surface shape, with optics (SO) which are rigid with respect to the object (O) being arranged in the object light path and following optics (BO), which move in the direction of their optical axis, after reflection of the object beam (OS),
**characterized**
**in that** the rigid optics (SO) are part of optics which produce an intermediate image of the object surface, and
**in that** the image sensor (BA) has image sensing elements (pixels), which are arranged in a planar form.

2. Measurement apparatus according to Claim 1,
**characterized**
**in that** the rigid optics (SO) have elements which shape the wavefront.

3. Measurement apparatus according to Claim 1 or 2,
**characterized**
**in that** the rigid optics (SO) are entirely or partially in the form of an endoscope.

4. Measurement apparatus according to one of the preceding claims,
**characterized**
**in that** the rigid optics (SO) image the object at infinity.

5. Measurement apparatus according to one of the preceding claims,
**characterized**
**in that** the moving optics (BO) are entirely or partially composed of optical elements which are mounted such that they can move on the optical axis.

6. Measurement apparatus according to one of the preceding claims,
**characterized**
**in that** an image of the reference plane (VR) is within the depth of focus range of the imaging optics.

7. Measurement apparatus according to Claim 6,
**characterized**
**in that** the image of the reference plane (VR) is on the image plane of the imaging optics.

8. Measurement apparatus according to Claim 6 or 7,
**characterized**
**in that** the image of the reference plane (VR) moves in synchronism with the image plane of the imaging optics during movement of the moving optics (BO).

9. Measurement apparatus according to one of the preceding claims,
**characterized**
**in that** the rigid optics (SO) are in the form of a rigid intermediate imaging apparatus (L2, L3), by means of which at least one intermediate image (SZB), which is rigid with respect to the object (O), of the object surface is produced, and
**in that** objective optics, which follow the rigid intermediate image (SZB) in the beam path, in the direction of their optical axis (ROA) are formed as the moving optics (BO), such that they move for scanning on the rigid intermediate image (SZB), which is aligned at right angles to this axis, in the depth direction (Z) and for imaging thereof directly or via one or more intermediate images on the image sensor (BA).

10. Measurement apparatus according to Claim 9,
**characterized**
**in that** the intermediate imaging apparatus (L2, L3) has an imaging scale which is the same for all of the object points which are imaged in the intermediate image (SZB).

11. Measurement apparatus according to Claim 10,
**characterized**
**in that** the intermediate imaging apparatus (L2, L3) is in the form of a telecentric imaging apparatus in a 4f arrangement.

12. Measurement apparatus according to one of the preceding claims,
**characterized**
**in that** optics (KSO) which correspond at least partially to the optics in the object light path are provided for compensation in the reference light path.

13. Measurement apparatus according to one of the preceding claims,
**characterized**
**in that** the position of the objective optics (BO) at which the highest interference contrast occurs is detected for each pixel.

14. Measurement apparatus according to one of Claims 9 to 13,
**characterized**
**in that** a deflection unit (AE) is provided, in order to produce the intermediate image (SZB), for a viewing direction of the intermediate imaging apparatus (L2, L3) which differs from the normal to the object surface.

15. Measurement apparatus according to one of the preceding claims,
**characterized**
**in that** a moving unit outside the moving objective optics (BO) comprises an illumination unit with the light source (L2) and the beam splitter (ST), or, outside the moving objective optics (BO), comprises only the beam splitter (ST).

16. Measurement apparatus according to one of Claims 9 to 15,
**characterized**
**in that** the rigid intermediate imaging apparatus (L2, L3) is in the form of an endoscope.

17. Measurement apparatus according to one of the preceding claims,
**characterized**
**in that** fibre optics (LL) are provided for illumination of the object (O) and of the reference plane (RSP).

18. Measurement apparatus according to one of the preceding claims,
**characterized**
**in that** the object light path and the reference light path as well as further light paths have as the lenses (L1, L2, L3, L4, KSO) individual lenses, grin (gradient-index) lenses, rod lenses, diffractive elements, prisms or their combinations.

19. Measurement apparatus according to one of the preceding claims,
**characterized**
**in that** an optical element (DE) for rotation of an image of the object (O) is located in the imaging optics within or outside the object light path, and is used to produce a fixed image of the object (O) on the image sensor (BA) when the rigid optics (SO) are rotated.

20. Measurement apparatus according to one of the preceding claims,
**characterized**
**in that** a folding endoscope (KL) is arranged as the rigid optics (SO) or as part of the rigid optics (SO) in the object light path and can be set to at least two folded positions, with an angle (α) between a folded-in state and a folded-out state.

21. Measurement apparatus according to Claim 20,
**characterized**
**in that** the folding endoscope (KL) has two tubes (T1, T2) which are connected to one another by means of a joint (G) and in which optical components (OKL) of the folding endoscope (KL) are accommodated, including a deflection element (KSP).

22. Measurement apparatus according to Claim 21,
**characterized**
**in that** a spring mechanism (F), which interacts with the two tubes (T1, T2), is arranged in the area of the joint (G).

## Revendications

1. Dispositif de mesure interférométrique (1) pour mesurer la forme d'une surface d'un objet (O) par interférométrie de lumière blanche, comprenant une source de rayonnement (LQ) émettant un rayonnement à cohérence courte, un diviseur de rayons (ST) pour former un rayon d'objet (OS) conduit par un trajet de lumière d'objet vers l'objet (O) et un rayon de référence (RS) conduit par un trajet de lumière de référence vers un plan de référence réfléchissant (RSP), et comprenant un récepteur d'images (BA) qui reçoit le rayonnement réfléchi par l'objet (O) et par le plan de référence (RSP) et mis en interférence, et le conduit vers un dispositif d'exploitation pour déterminer la forme de surface, avec dans le trajet de lumière d'objet une optique (SO) fixe par rapport à l'objet (O) qui est suivie d'une optique mobile (BO) en direction de son axe optique après la réflexion du rayon d'objet (OS),
**caractérisé en ce que**
l'optique fixe (SO) fait partie d'une optique générant une image intermédiaire de la surface d'objet, et le récepteur d'images (BA) présente des éléments de réception d'images (pixels) disposés de manière plane.

2. Dispositif de mesure selon la revendication 1,
**caractérisé en ce que**
l'optique fixe (SO) présente des éléments déformant le front d'ondes.

3. Dispositif de mesure selon la revendication 1 ou 2,
**caractérisé en ce que**
l'optique fixe (SO) présente entièrement ou partiellement la forme d'un endoscope.

4. Dispositif de mesure selon l'une quelconque des revendications précédentes,
**caractérisé en ce que**
l'optique fixe (SO) reproduit l'objet à l'infini.

5. Dispositif de mesure selon l'une quelconque des revendications précédentes,
**caractérisé en ce que**
l'optique mobile (BO) est entièrement ou partiellement constituée d'éléments optiques logés de manière mobile dans l'axe optique.

6. Dispositif de mesure selon l'une quelconque des revendications précédentes,
**caractérisé en ce qu'**
une image du plan de référence (VR) se situe dans la zone de profondeur de champ de l'optique de reproduction.

7. Dispositif de mesure selon la revendication 6,
**caractérisé en ce que**
l'image du plan de référence (VR) se situe dans le plan d'image de l'optique de reproduction.

8. Dispositif de mesure selon la revendication 6 ou 7,
**caractérisé en ce que**
lors d'un mouvement de l'optique mobile (BO) l'image du plan de référence (VR) se déplace de façon synchronisée avec le plan d'image de l'optique de reproduction.

9. Dispositif de mesure selon l'une quelconque des revendications précédentes,
**caractérisé en ce que**
l'optique fixe (SO) présente la forme d'un dispositif de reproduction intermédiaire (L2, L3) qui génère au moins une image intermédiaire (SZB) de la surface d'objet fixe par rapport à l'objet (O), et en tant qu'optique mobile (BO) une optique d'objectif est configurée dans le trajet de rayons en aval de l'image intermédiaire fixe (SZB), mobile en direction de son axe optique (ROA) pour balayer dans le sens de la profondeur (Z) l'image intermédiaire fixe (SZB) normalement alignée sur cet axe et pour reproduire celle-ci directement ou par l'intermédiaire d'une ou de plusieurs reproductions intermédiaires sur le récepteur d'images (BA).

10. Dispositif de mesure selon la revendication 9,
**caractérisé en ce que**
le dispositif de reproduction intermédiaire (L2, L3) présente une échelle de reproduction identique pour tous les points d'objet reproduits dans l'image intermédiaire (SZB).

11. Dispositif de mesure selon la revendication 10,
**caractérisé en ce que**
le dispositif de reproduction intermédiaire (L2, L3) présente la forme d'un dispositif de reproduction télécentrique selon une disposition en 4f.

12. Dispositif de mesure selon l'une quelconque des revendications précédentes,
**caractérisé en ce que**
pour la compensation une optique (KSO) est prévue dans le trajet de lumière de référence correspondant du moins en partie à l'optique dans le trajet de lumière d'objet.

13. Dispositif de mesure selon l'une quelconque des revendications précédentes,
**caractérisé en ce que**
pour chaque pixel on détecte la position de l'optique d'objectif (BO) pour laquelle se produit le contraste d'interférence maximum.

14. Dispositif de mesure selon l'une quelconque des revendications 9 à 13,
**caractérisé en ce que**
pour une direction de vue du dispositif de reproduction intermédiaire (L2, L3) s'écartant de la normale de la surface d'objet, une unité de déflection (AE) est prévue pour générer l'image intermédiaire (SZB).

15. Dispositif de mesure selon l'une quelconque des revendications précédentes,
**caractérisé en ce qu'**
une unité mobile comprend, outre l'optique d'objectif mobile (BO), une unité d'éclairage avec la source de lumière (L2) et le diviseur de rayons (ST), ou outre l'optique d'objectif mobile (BO), uniquement le diviseur de rayons (ST).

16. Dispositif de mesure selon l'une quelconque des revendications 9 à 15,
**caractérisé en ce que**
le dispositif de reproduction intermédiaire (L2, L3) présente la forme d'un endoscope.

17. Dispositif de mesure selon l'une quelconque des revendications précédentes,
**caractérisé en ce que**
pour l'éclairage de l'objet (O) et du plan de référence (RSP) une optique de fibres (LL) est prévue.

18. Dispositif de mesure selon l'une quelconque des revendications précédentes,
**caractérisé en ce que**
le trajet de lumière d'objet et le trajet de lumière de référence ainsi que d'autres trajets de lumière présentent en tant que lentilles (L1, L2, L3, L4, KSO) des lentilles uniques, des lentilles « Grin » (à indice de gradient), des lentilles à tige, des éléments diffractifs, des prismes ou des combinaisons de ceux-ci.

19. Dispositif de mesure selon l'une quelconque des revendications précédentes,
**caractérisé en ce que**
dans l'optique de reproduction, à l'intérieur ou à l'extérieur du trajet de lumière d'objet, est situé un élément optique (DE) pour faire tourner une image de l'objet (O) permettant d'obtenir une image fixe de l'objet (O) sur le récepteur d'images (BA) lorsque l'on fait tourner l'optique fixe (SO).

20. Dispositif de mesure selon l'une quelconque des revendications précédentes,
**caractérisé en ce que**
dans le trajet de lumière d'objet, comme optique fixe (SO) ou partie de l'objet fixe (SO) un endoscope pliant (KL) peut être réglé dans au moins deux positions avec un angle (a) entre un état plié et un état déplié.

21. Dispositif de mesure selon la revendication 20,
**caractérisé en ce que**
l'endoscope pliant (KL) présente deux tubes (T1, T2) reliés l'un à l'autre au moyen d'une articulation (G), dans lesquels sont logés des composants optiques (OKL) de l'endoscope pliant (KL) y compris un élément de déflexion (KSP).

22. Dispositif de mesure selon la revendication 21,
**caractérisé en ce que**
dans la zone de l'articulation (G) est disposé un mécanisme à ressort (F) coopérant avec les deux tubes (T1, T2).
